# EUROPEAN PATENT APPLICATION

(11) **EP 1 319 717 A1**
(43) Date of publication of application: **18.06.2003**
(21) Application number: 01129229.9
(22) Date of filing: 11.12.2001
(51) Int. Cl.: C12Q 1/68, C12Q 1/70

(54) **Method for detection of inflammatory processes**

(71) Applicant: Roche Diagnostics GmbH, 68305 Mannheim (DE); F. HOFFMANN-LA ROCHE AG, 4070 Basel (CH)
(72) Inventor: Meuer, Stefan, 69121 Heidelberg (DE); Giese, Thomas, 69198 Schriesheim (DE)

(57) **Abstract**

The present invention is based on the surprising observation, that a high degree of expression of certain distinct Genes in peripheral blood mononuclear cells is associated with in inflammatory process with a higher probability than it is the case for other Genes. Therefore, the present invention provides a method for diagnosis of an inflammatory process comprising monitoring expression levels of 4-6 selected targets in peripheral blood mononuclear cells as compared to expression levels of said selected targets in peripheral blood mononuclear cells in healthy individuals, characterized in that over-expression or repression of the majority of said selected Genes is indicative for an inflammatory process.

## Description

The present invention relates to the field of diagnosis of inflammatory processes. More precisely, the invention relates to the field of diagnosis of inflammatory processes by means of Cytokine expression profiling.

The following examples, references and figures are provided to aid the understanding of the present invention, the true scope of which is set forth in the appended claims. It is understood that modifications can be made in the procedures set forth without departing from the spirit of the invention.

### Prior art background

Inflammatory processes are locally restricted events characterized by the involvement of leucocytes (granulocytes, lymphocytes, and monocytes). Some of these cells are released into the blood stream such that from this source, the may easily be isolated and subjected to diagnostic approaches. For example, gene expression in peripheral blood mononuclear cells may be monitored, preferentially by highly sensitive methods such as RT-PCR.

A variety of genes encoding different types of proteins are involved in inflammatory processes are cytokines and especially chemokines . Expression of these proteins is a major focus in biomedical research because of their numerous functions and disease associations. Direct evaluation of these mediators as proteins by enzyme immunoassay, immunohistochemistry or flow cytometry requires specific antibodies and assay sensitivity is variable. Since cytokine gene expression (mRNA) is usually quantitatively correlated to the level of produced protein, RT-PCR is widely used as an alternative method to describe cytokine profiles. Quantification of mRNA does not require the accumulation of measurable amounts of protein and hence is a more sensitive and dynamic monitor of gene expression.

Besides chemokines, expression of other proteins is affected during inflammation. For example, expression of genes encoding transcription factors has also been shown to be altered during an inflammatory process.

Although highly sensitive, conventional RT-PCR is labor intensive and difficult to use for quantification. Many semi-quantitative RT-PCR methods that use external standards and competitors are available. However, all require considerable time and effort. Amplification is usually followed by manual gel analysis and band intensities are compared visually or by densitometry.

Many of the difficulties of conventional semi-quantitative RT-PCR are overcome with the newly available LightCycler system. Continuous detection of accumulating PCR product each cycle allows rapid and reliable quantification of gene expression

It is known for a long time, that allograft rejection events correlated with severe inflammatory processes (14, 15, 16, 19). Diagnosis of allograft rejections conventionally relies on histopathological examination of biopsies, like e.g. endomyocardial biopsies in case of a cardiac allograft. As an alternative, predictive diagnosis of an allograft rejection event may be based on quantitative gene expression analysis of genes which are overexpressed during an inflammatory process (1,2,3,4,11).

Since, however, biopsy sampling is an invasive technique, it implies a certain risk of discomfort on the patient, may not repeated deliberately, and causes considerable costs. Although routinely used in clinical practice, histopathological rejection gradings on the basis of round cell infiltration suffers from several limitations. First, the degree of round cell infiltration may differ regionally and not be reflected in a small biopsy sample, questioning the limited number of biopsies. Furthermore, the quality of a histopathological diagnosis is depending on the operator and in addition reveals some inter-observer variability. Finally, prediction of functional phenomena based on morphological findings carries considerably inaccuracy.

Recently, it has been suggested to replace analysis of biopsies by analysis of peripheral blood mononuclear cells. In this context, several publications have discussed the possibility of a correlation of expression of single or a few Genes or Cytokines in peripheral blood mononuclear cells with a defined inflammatory process like a transplantation event. However, with regard to putative clinical applications, all attempts of analyzing only a small number of genes resulted in high percentage of false negative and false positive results, independently from the target genes chosen (6,8,12).

It has also been suggested to diagnose inflammatory processes especially associated with renal allograft rejection based on a method which monitors a larger number of arbitrarily chosen genes (2) but the available data showed only poor to moderate correlation between the determined expression differences and the severity of the inflammatory process.

Moreover, infection with Hepatitis C virus (HCV) is one of the leading causes of liver failure worldwide. The pathogenesis of progressive HCV liver disease involves both inflammation and fibrosis, which requires a diagnosis as early as possible.

Summarizing all methods known in the art, have either the disadvantage of invasive sample preparation techniques combined with only qualitative histopathological examinations, or alternatively, if based on expression analysis a single gene or a number of arbitrarily chosen genes, being not very reliable.

Thus, there is a need in the art to provide an improved method for diagnosis of an inflammatory process with a more reliable diagnostic value.

### Brief description of the invention

The present invention provides a method for diagnosis of an inflammatory process comprising monitoring expression levels of 4-6 selected Genes in peripheral blood mononuclear cells as compared to expression levels of said selected Genes in peripheral blood mononuclear cells in healthy individuals, characterized in that a 50% difference in expression of at least 50% of said selected Genes is indicative for an inflammatory process. Wherein expression of at least one of said selected genes is repressed during inflammatory processes.

Similarly, the present invention also provides a method for diagnosis of an inflammatory process comprising monitoring expression levels of 4-6 selected Genes in peripheral blood mononuclear cells as compared to expression levels of said selected Genes in peripheral blood mononuclear cells in healthy individuals, characterized in that for each of said selected Genes, a scoring value corresponding to the degree of difference in expression as compared to normal cells is determined, and further characterized in that the overall value calculated as the sum of all determined scoring values is indicative for the probability of an inflammatory process, with the provisio, that at least one gene usually under expressed in inflammatory genes is monitored.

Preferentially, the first and second selected Cytokines are MRP-14 and/or Interleukin-6 and/or Interleukin-8. It is also advantageous, if further selected Genes are Interferon-gamma and/or Perforin.

Most conveniently, the expression levels of the selected genes are determined by means of RT-PCR, for example Real Time RT-PCR using the LightCycler instrument (Roche Molecular Biochemicals).

The inventive method may be used for diagnosis of a variety of inflammatory processes including allograft rejections.

In addition, the inventive method may be used for diagnosis of infection associated organic failures like, for example, diagnosis of an HCV associated hepatic failure.

A still further aspect of the invention is directed to a Kit for monitoring expression of 4-6 genes comprising reagents for amplifying sequences of MRP-14, Interleukin-6, Interferon-gamma and Perforin.

### Detailed description of the invention

The present invention is based on the surprising observation, that a high degree of difference in expression of certain distinct Cytokines and other Genes in peripheral blood mononuclear cells (PBMCs) is associated with an inflammatory process with a higher probability than it is the case for other Genes or Cytokines.

A variety of different cytokine and chemokine parameters has been disclosed in the past to be up-regulated during inflammatory processes. Analysis of single parameter expression for diagnostic purposes, however, to the best knowledge of the inventors has failed so far. In contrast, through screening of a great number of different clinical specimens, it was found that it is insufficient to correlate the expression levels of single parameters, because many cases can be identified wherein despite the existence of an inflammatory process caused by a local disease event, an up-regulation of a specific gene can not be observed.

Instead, it turned that a significant increase in the overall expression pattern of all cytokines and chemokine seems to be better correlated with an inflammatory processUnexpectedly, however, it has been proven by the inventors and is disclosed in the present invention, that there exist several genes the expression of which seems to be down-regulated with reasonable statistical occurrence at the beginning or during an inflammatory process, and, moreover, that quantitative monitoring of down-regulation may contribute to a reliable method for diagnosing an inflammatory process.

Most of these genes are either cytokines or transcription factors.

Even more unexpectedly, it has been proven by the inventors that although there exits no possibility to significantly correlate expression of a single parameter with the existence of an inflammatory process for diagnostic purposes, it is sufficient to analyze only a small number of parameters, provided that the parameters are carefully selected. In other words, over-expression or repression in the majority of a set of several specifically selected parameters is significant for diagnosis of an inflammatory process, whereas over-expression or repression in the majority of a set of several arbitrarily selected parameters is not.

Consequently, in a first aspect, the present invention provides a method for early diagnosis of an inflammatory process comprising monitoring expression levels of 4-6 selected genes in peripheral blood mononuclear cells as compared to expression levels of said selected genes in peripheral blood mononuclear cells in healthy individuals, characterized in that a 50% difference in expression of at least 50% of said selected genes is indicative for an inflammatory process. In this context, a 50 % difference in expression is defined as being either a twofold over expression of, alternatively a 50 % repression as compared to the average expression level in peripheral blood mononuclear cells of a healthy individual.

In order to reach a higher level of specificity thus reducing the sensitivity of the inventive method, it is preferred if more than 60% or even more preferable 75% of said selected parameters are either over-expressed twofold or repressed to a residual level of less than 50%.

Similarly, the present invention also provides a method for diagnosis of an inflammatory process comprising monitoring expression levels of 4-6 selected genes in peripheral blood mononuclear cells as compared to expression levels of said selected genes in peripheral blood mononuclear cells in healthy individuals, characterized in that for each of said selected Genes, a scoring value corresponding to the degree of difference in expression of is determined, and further characterized in that the overall value calculated as the sum of all determined scoring values is indicative for the probability of an inflammatory process. In this context, scoring is done in such a way that positive scoring values are given corresponding to the absolute difference in expression, regardless of whether the difference is an increase or a decrease.

It is highly preferable, if of said selected parameters is MRP-14., since this parameter is over-expressed in case of an inflammatory process with the highest specificity.

MRP-14 (Calgranulin B) is a calcium-binding protein of the S100 family. This protein is expressed during myeloid differentiation, is abundant in granulocytes and monocytes, and forms with MRP-8 a heterodimeric complex in a Ca2+-dependent manner. Phagocytes expressing MRP8 and MRP14 belong to the early infiltrating cells and dominate acute inflammatory lesions. (20).

Interleukin-6 (IL-6) is a pleiotropic cytokine that acts on a wide variety of cell types. It has important regulatory functions in the immune system, is a mediator of the acute-phase response, and is involved in the regulation of differentiation, proliferation, and survival of target cells. (21)

IFN-gamma is produced mainly by T-cells and natural killer cells activated by antigens, mitogens, or alloantigens. IFN-gamma has antiviral and antiparasitic activities and also inhibits the proliferation of a number of normal and transformed cells. The main biological activity of IFN-gamma appears to be immunomodulatory in contrast to the other interferons which are mainly antiviral. (22)

Perforin is a cytolytic protein that make membranes permeable by polymerizing in the presence of calcium and by forming transmembrane channels. Perforin belongs to the major factors responsible for the cytotoxic activities of lymphocytes. (23)

The inventive method may be used for diagnosis of a variety of inflammatory processes including allograft rejections.

In one embodiment, the inventive method may be used for early detection of allograft rejection events. For example, but not limiting the invention, the new method may detect kidney allograft rejection (see example 3), cardiac allograft rejection or liver allograft rejection events, which are all known to be based on an inflammatory process. In this context it is important to note that although transplant rejections represent a local immune reaction, alloreactive activated effector cells have been shown to be present at low frequency in the blood stream.

According to the invention Multi-gene expression analysis in BPMCs, provided that genes are selected appropriately according to the invention, in contrast to histopathology, generates information on the functional status of effector cells. Thus, besides detecting clinically relevant rejection incidents, the new invention is also useful to adjust immunosuppressive treatment to the respective individual requirements.

In another aspect, the invention provides a possibility for sensitive detection of an immunopathogenic process in HCV-infected patients by means of monitoring multigene expression in PBMC, if genes are selected appropriately. Thus, due to the high correlation between expression of selected genes in PBMCs and observed hisptopathologic changes the new method provides a possibility to detect an HCV induced systemic disease process including a severe hepatic failure. This is especially valuable because usually, severity of such a systemic disease process does not correlate with viral load of the infected patient.

Gene expression may be monitored in many different ways. For example, expression of Cytokine and Chemokine proteins may be measured in a semiquantitative manner by methods known in the art such as gel electrophoresis, or Protein array techniques. Quantitative results are preferably obtained using respective ELISA assays.

Expression of Cytokines and other Genes is especially monitored on the RNA level, for example applying conventional techniques like Northern blot analysis, or RNAse protection assays or nucleic acid array technologies.

Most conveniently, the expression levels of the selected Genes are determined by means of RT-PCR, for example real time RT-PCR, since this techniques are more sensitive and highly reproducible. Due to its extraordinary possibility to generate quantitative results, it is mostly preferred to perform real-time RT-PCR, like it is disclosed for example in US 6,174,670.

Sample preparation is one of the most critical aspects of quantitative PCR of PBMCs, since isolation of high quality RNA is an important first step for the quantification of gene expression. This step has to be easy, quick and reliable. For high sample throughput a microformat is preferred.

It has also been proven to be advantageous in case an automated system for sample preparation is used. Reliable results, for example, may be obtained using the MagnaPure Nucleic acid sample preparation system (Roche Molecular Biochemicals).

Total cellular RNA is sufficient for analysis but contamination of DNA should be minimal. RNA sequences to be amplified may not only be derived from total cellular RNA but also from mRNA which may have been purified by any kind of purification known in the art.

This includes but is not limited to purification protocols using Guanidinium and phenol (24) or quick chromatographic procedures like High Pure spin columns (Roche Molecular Biochemicals)

RNA has to be transcribed into first strand cDNA. Synthesis of cDNA can be performed according to different protocols, wherein first strand and second strand synthesis are either performed by two different enzymes or by using the same enzyme for both steps. For the first strand synthesis, any kind or RNA dependent DNA Polymerases (reverse transcriptase) may be used, e.g. AMV Reverse Tanscriptase, MMLV Reverse Transcriptase or mutants thereof or thermoactive DNA polymerases like C.therm or Tth (Roche Molecular Biochemicals). Subsequently, the RNA/cDNA hybrid is subjected to thermal denaturation or alternatively, the RNA template within the hybrid may be digested by RnaseH. Second strand synthesis may then be performed with any kind of DNA dependent DNA Polymerase, e.g. Klenow DNA Polymerase or Taq DNA Polymerase.

For an approach where first and second strand synthesis is done with one enzyme , Thermus thermophilus or C.Therm (Roche Diagnostics) may be used. Using the one-step or one-tube RT-PCR reduces handling and time, but requires separate RT-reactions for every parameter studied and housekeeping gene used to normalize sample concentration.

In the two step system, first strand synthesis is performed in a separate reaction for all parameters simultaneously, followed by parameter-specific PCR analysis. Usually cDNA obtained from 1 µg total RNA is sufficient to analyze 50 parameters. Therefore, in the context of performing a method according to the invention, the two step system is highly preferred. Good and reliable results are especially obtained with a two step system using total RNA isolation and first strand cDNA synthesis with AMV.

Although usually not necessary for PCR based methods, it may be in some cases advantageous in at least some cases, if the double stranded cDNA is subsequently being purified prior to amplification. Purification may be achieved by means of conventional precipitation, Phenol/Chloroform extraction, using Sephadex spin coloumns, HighPure spin coloumns (Roche Molecular Biochemicals) or any other method known in the art.

Amplification of the double stranded cDNA is preferably performed by means of PCR using a thermostable DNA polymerase and two amplification primers which are completely or at least substantially complementary to the primer binding sites introduced by the first and the second oligonucleotide, respectively. Due to the general knowledge on PCR, a person skilled in the art will be capable of establishing suitable PCR conditions for effective high yield amplification through routine development procedures. In addition, other amplification methods, like e.g. NASBA (25) may also be applied.

For subsequent expression studies on nucleic acid arrays, the amplified cDNA may become in vitro transcribed in an additional step by methods well known in the art (26). In order to enable transcription, the first extendible primer comprises a promoter for in vitro transcription. It is essential that the promoter is located downstream of the segment which by itself is capable of serving as a primer binding site for nucleic acid amplification primers. In addition it is required that the promoter is located upstream of the segment which is capable of hybridizing to substantially all RNAs contained in the pool to become amplified. In other words: the promoter is preferably located between said first and said second segment.

It is specifically preferred, however, if expression is monitored by means of real time RT-PCR. Real time PCR and especially real time RT-PCR may be performed with a variety of different alternative detection formats, among which the most established ones are:
a) FRET hybridization probes
   For this test format two single-stranded hybridization probes are used simultaneously which are complementary to adjacent sites of the same strand of the amplified target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured when both hybridization probes bind to adjacent positions of the target molecule to be detected.
   Among all detection formats possible within the scope of the present invention, this "FRET-hybridization probe" has been proven to be highly sensitive, exact and reliable.
   Alternatively, it is also possible to use a fluorescent-labeled primer and only one labeled oligonucleotide probe (27).
b) TaqMan hybridization probes
   A single-stranded hybridization probe is labeled with two components. When the first component is excited with light of a suitable wavelength, the absorbed energy is transferred to the second component, the so-called quencher, according to the principle of fluorescence resonance energy transfer. During the annealing step of the PCR reaction, the hybridization probe binds to the target DNA and is degraded by the 5'-3' exonuclease activity of the Taq Polymerase during the subsequent elongation phase. As a result the excited fluorescent component and the quencher are spatially separated from one another and thus a fluorescence emission of the first component can be measured (US 5,210,015).
c) Molecular Beacons
   These hybridization probes are also labeled with a first component and with a quencher, the labels preferably being located at both ends of the probe. As a result of the secondary structure of the probe, both components are in spatial vicinity in solution. After hybridization to the target nucleic acids both components are separated from one another such that after excitation with light of a suitable wavelength the fluorescence emission of the first component can be measured (US 5,118,801).
d) SybrGreen format
   It is also within the scope of the invention, if real time PCR is performed in the presence of an additive according to the invention in case the amplification product is detected using a double stranded nucleic acid binding moiety. For example, the respective amplification product can also be detected according to the invention by a fluorescent DNA binding dye which emits a corresponding fluorescence signal upon interaction with the double-stranded nucleic acid after excitation with light of a suitable wavelength. The dyes SybrGreen and SybrGold (Molecular Probes) have proven to be particularly suitable for this application. Intercalating dyes can alternatively be used. However, for this format, in order to discriminate the different amplification products, it is necessary to perform a respective melting curve analysis (US 6,174,670).

Another important feature for performing assays according to the invention is a good amplification primer design. The amplicon should be smaller than 600 bp (optimal between 250 and 350 bp) and no genomic sequences should be amplified. This is verified by PCR "with no RT" controls (cDNA reaction in the absence of reverse transcriptase) and / or with genomic DNA. Genomic products above 1500 bp are usually not amplified in the LightCycler using standard rapid cycling protocols.

For the SybrGreenI format, it is important, that only one specific product is amplified. Amplification primers should have a high threshold for mispriming. Searching different databases with the sequence of the selected primer can be very helpful. Increasing the stringency of the amplification (e.g. touchdown PCR) can also enhance the specific amplification of a target. A computer aided search for primer sequences with highly stringent binding properties is recommended. For example, primers may be selected with the aid of Oligo primer analysis software.

Regardless of the actual method of performing RT-PCR which is chosen, it is highly advantageous, to use method of relative quantification of gene expression. Therefore, it is within the scope of the invention if internal housekeeping gene levels are monitored in order to normalize the degree of inflammatory gene expression.

Summarizing, it is emphasized that the new method provides a non-invasive diagnostic tool for analysis of inflammation processes associated with local disease events, which in many different aspects may replace conventional methods based on analysis of biopsy material.

### Description of the Figures

**Figure 1:**
   Plot showing average expression of 36 different Genes in peripheral blood mononuclear cells from 6 kidney allograft rejection patients as compared to the expression of 4 kidney allograft patients without rejection.
**Figure 2:**
   Multigene expression score of 6 kidney allograft rejection patients as compared to the expression of 4 kidney allograft patients without rejection, based on expression of certain Genes selected according to the invention. Details are disclosed in the examples.
**Figure 3:**
   Density gradient centrifugation according to example 1

### Examples

The following examples, references, sequence listing and figures are provided to aid the understanding of the present invention, the true scope of which is set forth in the appended claims. It is understood that modifications can be made in the procedures set forth without departing from the spirit of the invention.

### Example 1: Automated sample preparation and reverse transcription

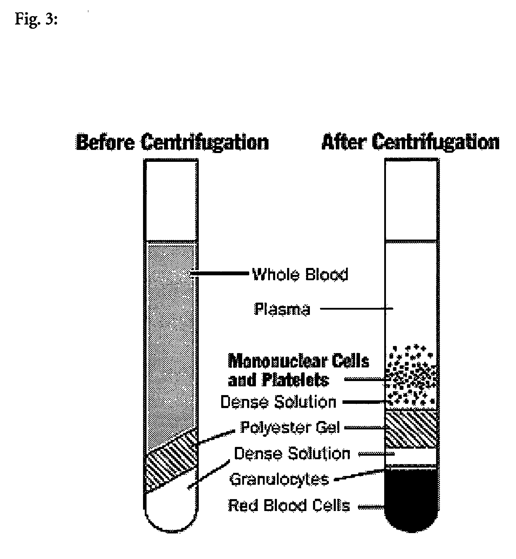

Peripheral blood was collected into CPT-Vacutainer (Becton Dickinson). Centrifugation of the CPT tube resulted in dividing the contents into four distinct layers (Figure 3):
- Packed red cells, granulocytes and "Dense Solution" at the bottom of the tube
- Inert plug separating RBC's from Peripheral Blood mononuclear Cells (PBMC)
- Ficol ("Dense Solution") layer containing PBMC's
- Plasma

The PBMC layer was carefully transfered to a 15 ml falcon tube and washed three times with cell culture medium. 2 * 10⁶ PBMC were cultured in 1 ml RPMI 1640 with 10% FCS for 3 hr at 37°C to regain basic transcription levels. After lysis in 300 µl MagNAPure lysis puffer the sample were frozen at -70°C. After thawing the lysates were well mixed and transferred into the MagnaPure samples cartridge and mRNA was isolated with the MagnaPure-LC device using the mRNA standard protocol for cells. The elution volume was set to 50µl. Two aliquots of 8.2 µl RNA were independent reverse transcribed using AMV-RT and oligo- (dT) as primer (First Strand cDNA synthesis kit, Roche) according to the manufactures protocol in a thermocycler. After termination of the cDNA synthesis, the reaction mix was diluted to a final volume of 500 µl and stored at -20°C until PCR analysis.

### Example 2: Manual sample preparation and reverse transcription

Mononuclear cells (MNC) were isolated on a Histopaque 1077 density gradient using Leuco Sep tubes (Greiner Labortechnik Frickenhausen, Germany) and 2x10⁶ cells were resuspended in RPMI 1640 with 10 % FCS. Cultures were stimulated with 10 ng/ml PMA and 0.5 µg/ml Ionomycin for various time points at 37 °C in 7 % CO₂. Cells were harvested, resuspended in 200 µl PBS and 400 µl of High-Pure lysis solution was added. Resulting lysates were stored at -70 °C. After thawing at 37 °C for 10 min, RNA was extracted (RNA isolation kit) and eluted from the spin column in a volume of 50 µl. An aliquot of 8.2 µl RNA was reverse transcribed using AMV-RT and oligo-(dT) as primer (cDNA synthesis kit).

### Example 3: Quantitative Real Time hot start PCR using the SybrGreen detection modus

Target sequences were amplified using commercially available LightCycler Primer Sets (Search-LC, Heidelberg) with the LightCycler FastStart DNA Sybr Green I Kit (Roche Diagnostics) according to the manufactures protocol. Briefly, a reaction mix of 2µl FastStart DNA SybrGreen I mastermix, 2 µl of the Primer set and 6 µl of PCR grade water were premixed and added to a LightCycler capillary together with 10 µl of the diluted cDNA sample. After brief centrifugation, the capillaries were placed into the thermal chamber of the LightCycler and the polymerase was activated for 10 minutes at 95 °C. Denaturation was set to 95°C for 10 seconds. Annealing was performed with touchdown technique: starting temperature 68°C; decrease of 0.5 °C per cycle over 20 cycles for 10 seconds and elongation occurred at 72°C for 16 seconds. PCR was performed for 35 cycles.

The copy number was calculated from a virtual standard curve, obtained by plotting a known input concentration of a plasmid to the PCR-cycle number (CP) at which the detected fluorescence intensity reaches a fixed value.

RNA input was normalized by the average expression of the two housekeeping genes β-Actin and Cyclophilin B in healthy donors. Since this factor remained constant, all samples are comparable with each other.

The data of the two independent analyses for each sample and parameter were averaged and presented as adjusted transcripts / µl cDNA. Multiplying these values by 3000 would generate the transcript number per sample.

### Example 4: Expression analysis of Cytokines and Genes in kidney allograft patients

Expression of 36 different Cytokines and Genes in PBMCs from 6 kidney allograft patients suffering from an allograft rejection was monitored and quantified according to example 3. Primers were designed using the "Oligo Primer Analysis" software (MBI.)

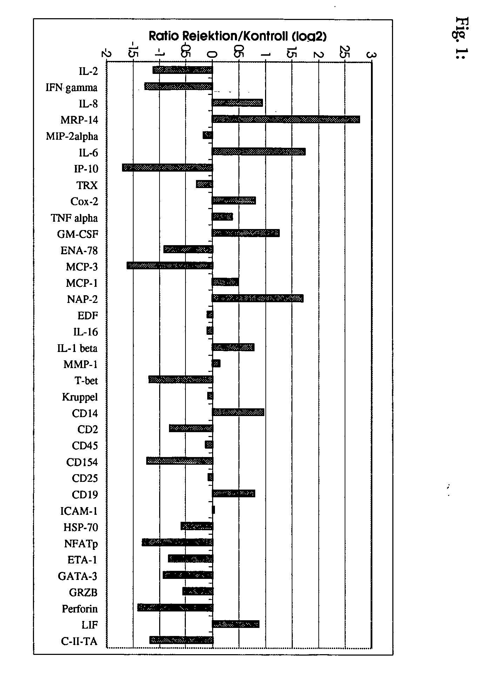

The results are indicated in figure 1. More precisely, this plot indicates the log2 of the average ratio of expression of the 36 selected parameters in the patient collective of 6 individuals with allograft rejections as compared to a collective of 4 healthy individuals without an allograft rejection event.

As can be seen in the figure, there are certain genes like MRP-14 with are highly over-expressed in WBC from allograft rejection patients, whereas expression of other genes like e.g. IP-10 is strongly repressed. In this context, however, it is important to note that the values obtained for some of the parameters such as Il-6 an MCP-3 have shown high values of standard deviations and thus seem to be less relevant from a statistical point of view.

### Example 5: Multi gene expression scoring of kidney allograft rejection patients and kidney allograft patients without rejection

On the basis of the results obtained in example 3, a multigene expression score was established. Score-ranking was done as follows:

### MRP-14

0 = up to 3 fold
1 = up to 5-fold
2 = up to 10-fold
3 = over 10-fold increase

### IL-8

0 = up to 1,5 fold
1 = up to 3-fold
2 = up to 10-fold
3 = over 10-fold increase

### IFN-gamma, Perforin, C-II-TA, CD154

0 = up to 30 %
1 = up to 70 %
2 = up to 90 %
3 = over 90% decrease

Based on this scoring system, expression of the selected Genes was plotted for 6 renal transplantation patients with kidney allograft rejections and 4 renal allograft patients without an allograft rejection.

Expression in this particular case was monitored according to example 2 amplifying the following targets:

| Gene | Gene bank Accession No. | Nucleotide Position |
|---|---|---|
| MRP-14 | X06233 | 9-345 |
| IL-8 | XM_003501 | 101-366 |
| IFN-gamma | XM_006883 | 243-550 |
| Perforin | M28393 | 386-631 |
| C-II-TA | NM_000246 | 2993-3257 |
| CD154 | X67878 | 60-444 |

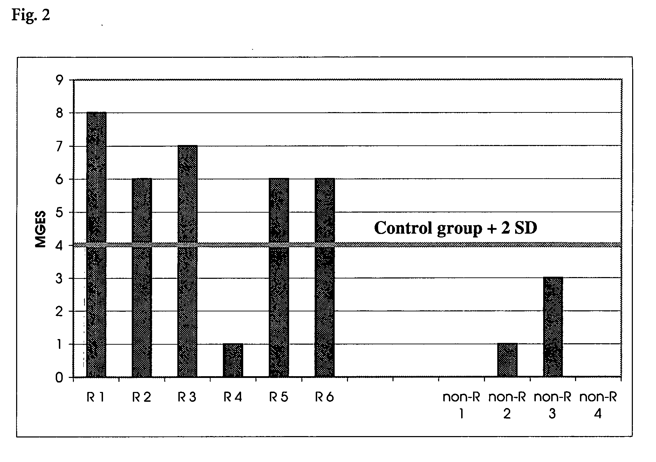

Results are shown in figure 2. As can be seen in the figure, a significant difference in scoring values was obtained for 5 of 6 renal allograft rejection patients as compared to renal allograft patients without an allograft rejection. More precisely, scoring values obtained for said 5 patients with a rejection event were significantly higher than the 2 fold standard deviation obtained for the scoring values of the control group. Thus, such a scoring method is applicable for early diagnosis of an inflammatory process which is indicative for an upcoming allograft rejection event.

### List of References

Akalin, E., et al., Transplantation 72 (2001) 948-53
Bernard, P. S., et al., Anal Biochem 255 (1998) 101-7
Chomczynski, P. and Sacchi, N., Anal Biochem 162 (1987) 156-9
de Groot-Kruseman, H. A., et al., J Heart Lung Transplant 20 (2001) 503-10
Dugre, F. J., et al., Transplantation 70 (2000) 1074-80
Hayry, P., Transplant Proc 28 (1996) 7-10
Hayry, P., et al., Immunol Rev 77 (1984) 85-142
Horn, F., et al., Immunobiology 202 (2000) 151-67
Ijzermans, J. N. and Marquet, R. L., Immunobiology 179 (1989) 456-73
Kerkhoff, C., et al., Biochim Biophys Acta 1448 (1998) 200-11
Kievits, T., et al., J Virol Methods 35 (1991) 273-86
Lagoo, A. S., et al., J Heart Lung Transplant 15 (1996) 206-17
Lemstrom, K., et al., Kidney Int Suppl 52 (1995) S2-10
Melter, M., et al., Pediatr Transplant 3 (1999) 10-21
Milligan, J. F., et al., Nucleic Acids Res 15 (1987) 8783-98
Moller, E., Transplant Proc 27 (1995) 24-7
Nickel, P., et al., Transplantation 72 (2001) 1158-60
Oh, S. I., et al., Transplantation 71 (2001) 906-9
Orosz, C. G. and VanBuskirk, A. M., Transplant Proc 30 (1998) 859-61
Pirenne, J., et al., Nucl Med Biol 21 (1994) 545-55
Shulzhenko, N., et al., Braz J Med Biol Res 34 (2001) 779-84
Shulzhenko, N., et al., Hum Immunol 62 (2001) 342-7
Stepp, S. E., et al., Immunol Today 21 (2000) 254-6
Strehlau, J., et al., Proc Natl Acad Sci U S A 94 (1997) 695-700
Strom, T. B. and Suthanthiran, M., Semin Nephrol 20 (2000) 103-7
Suthanthiran, M., Am J Med Sci 313 (1997) 264-7
van Emmerik, N., et al., Transpl Int 7 (1994) S623-6

US 5,118,801
US 5,210,015
US 6,174,670

## Claims

1. Method for diagnosis of an inflammatory process comprising monitoring expression levels of 4-6 selected genes in peripheral blood mononuclear cells as compared to expression levels of said selected Genes in peripheral blood mononuclear cells in healthy individuals,
**characterized in that** a 50% difference in expression of at least 50% of said selected genes is indicative for an inflammatory process,
wherein expression of at least one selected gene is decreased.

2. Method for diagnosis of an inflammatory process comprising monitoring expression levels of 4-6 selected genes in peripheral blood mononuclear cells as compared to expression levels of said selected genes in peripheral blood mononuclear cells in healthy individuals,
**characterized in that** for each of said selected genes, a scoring value corresponding to the degree of difference in expression of said selected genes is determined,
further **characterized in that** the overall value calculated as the sum of all determined scoring values is indicative for the probability of an inflammatory process,
wherein expression of at least one of said selected genes is decreased.

3. Method according to claims 1-2, wherein at least one of said selected genes is either MRP-14 or Interleukin-6.

4. Method according to claim 3, wherein at least one of said selected genes is either Interferon-gamma or Perforin.

5. Method according to claims 1-5, wherein said expression levels are determined by means of RT-PCR and preferentially real-time RT-PCR.

6. Use of a method according to claims 1-6 for diagnosis of an allograft rejection.

7. Use according to claim 7, wherein the allograft is a kidney allograft.

8. Use of a method according to claims 1-6 for diagnosis of a infection associated organic failure and especially an HCV associated hepatic failure.

9. Kit for monitoring expression of 4-6 genes comprising reagents for amplifying sequences of MRP-14, Interleukin-6, Interferon-gamma and Perforin.
